# EUROPEAN PATENT APPLICATION

(11) **EP 3 514 141 A1**
(43) Date of publication of application: **24.07.2019**
(21) Application number: 18305042.6
(22) Date of filing: 19.01.2018
(51) Int. Cl.: C07C 227/18, C07C 229/16, C07F 5/00

(54) **MAGNETIC MOLECULES**

(71) Applicant: Centre National de la Recherche Scientifique, 75016 Paris (FR); Université de Strasbourg, 67000 Strasbourg (FR)
(72) Inventor: HERMANS, Thomas, 67000 STRASBOURG (FR); SORRENTI, Alessandro, 8049, Zürich (CH)
(74) Representative: Lavoix

(57) **Abstract**

The invention relates to a composition comprising an oil and at least one molecule having the following general structure (I): 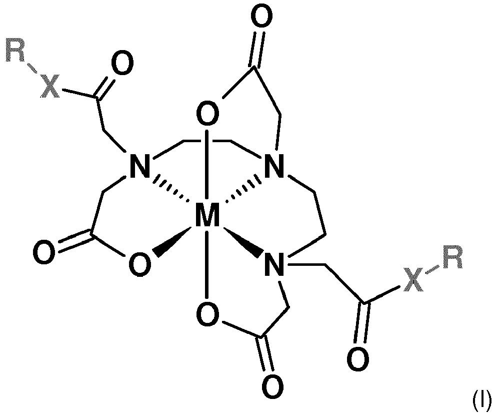 wherein X is O, N, or S ; R represents at each occurrence the same or different and independently linear or branched, saturated or unsaturated aliphatic chain, non-fluorinated, partially fluorinated or perfluorinated; and M is a trivalent paramagnetic ion.

The invention also relates to preparation and applications thereof.

## Description

The present invention relates to magnetic molecules, compositions comprising the same and in particular magnetic oils, their preparation and their applications.

Magnetic fields are already exploited in many areas of research and industrial practice to achieve levitation (e.g. high-speed bearings, maglev trains), but more importantly in magnetic separation. The latter operates from extremely large industrial scales (e.g., sorting scrap metal) down to removal of small (bio)molecules from biological fluids like blood. These separation methods make use of the magnetic field gradient force and the difference in susceptibility of material that is attracted to the magnet and its surrounding medium. In high (magnetic)liquids with very high susceptibility such as ferrofluids, a magnetic field can induce a spectacular structuring of the liquid, magnetic tuning of viscosity, or actuation. These phenomena not only work for magnetic liquids based on (super)paramagnetic particles, but also for lower magnetic susceptibility liquids containing paramagnetic ions.

In 2009, Coey *et al.* (Coey, J. M. D.; Aogaki, R.; Byrne, F.; Stamenov, P. Proc. Natl. Acad. Sci. USA 2009, 106 (22), 8811-8817) demonstrated proof-of-principle experiments that aimed to replace the conventional physical walls in fluidic devices with virtual walls by using specific magnetic field arrangements. Also, the European patent application filed as EP 17305070.9 on January 23, 2017 relates in particular to devices and methods for circulating a less-paramagnetic liquid into an enclosing more-paramagnetic liquid or for circulating a more-paramagnetic liquid into an enclosing less-paramagnetic liquid.

Complexes of paramagnetic ions such as lanthanides (e.g. Gd³⁺) with the chelating agents diethylenetriaminepentaacetic acid (DTPA) and 1,4,7,10-tetraazacyclododecane-1,4,7,10-tetraacetic acid (DOTA) are widely used as contrast agents in magnetic resonance imaging (MRI). These chelates are water soluble and provide the paramagnetic ion in a non-toxic (or less-toxic) form since the ion is kept in a stable coordination environment, which makes it difficult to be released.

Beside the classical low molecular weight MRI agents, a variety of amphiphilic (lipophilic) gadolinium complexes (i.e. containing the chelating group bound to a hydrophobic moiety) have been synthesised and characterised. These amphiphilic chelates self-assemble in aqueous media giving colloidally stable solutions of supramolecular aggregates such as micelles and liposomes. Alternatively, they have been used in mixture with one or more commercial lipids to form mixed aggregates by co-assembly. Finally, supramolecular aggregates of amphiphilic gadolinium molecules have been derivatized with specific peptides or antibodies as target specific MRI agents. The general aim of developing lipophilic gadolinium complexes is obtaining multimeric contrast agents with improved relaxivity, pharmacokinetic, biodistribution and targeting ability. All of the above examples comprise water soluble or water dispersable agents.

### Aims of the invention

The present invention aims to overcome the technical problem of developing new paramagnetic media and in particular paramagnetic oils.

The present invention aims to overcome the technical problem of providing molecules having paramagnetic properties, and in particular water-immiscible molecules.

In particular, the present invention aims to overcome the technical problem of providing molecules and compositions for use as paramagnetic media and in particular as paramagnetic oils.

The invention aims in particular to overcome the technical problem of providing paramagnetic media stable with a paramagnetic ion, in particular stable with a paramagnetic trivalent ion.

The present invention aims to overcome the technical problem of providing molecules which are miscible in a medium resulting in a water-immiscible fluid.

In particular, the present invention aims to overcome the technical problem of providing molecules useful for preparing non-ionic fluids, preferably with a positive magnetic susceptibility.

The present invention aims also to overcome the technical problem of providing molecules useful in transparent media.

The present invention also aims to overcome the technical problem of providing molecules useful in fluids stable in contact with water.

In particular, the present invention aims to overcome the technical problem of providing molecules useful for preparing paramagnetic fluids, in particular useful for methods and devices for circulating a less-paramagnetic liquid into an enclosing more-paramagnetic liquid or for circulating a more-paramagnetic liquid into an enclosing less-paramagnetic liquid, in particular as described in the European patent application EP 17305070.9.

The present invention aims in particular to overcome one or more of the above-recited technical problems.

The present invention aims to provide an industrial solution to one or more of the above-recited technical problems.

### Description of the invention

It has been discovered by the inventors that molecules and compositions according to the present invention overcome one or more of the above-recited technical problems. In particular, the present invention relates to a composition comprising an oil and at least one molecule having the following general structure (I): 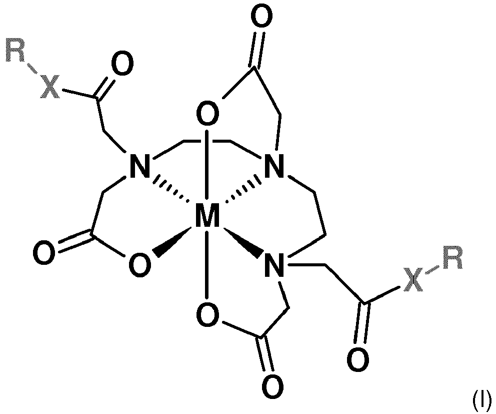 wherein X is O, N, or S ; R represents at each occurrence the same or different and independently linear or branched, saturated or unsaturated aliphatic chain, non-fluorinated, partially fluorinated or perfluorinated; and M is a trivalent paramagnetic ion.

In one embodiment, R comprises from 3 to 30 carbon atoms.

In one embodiment, R comprises from 6 to 30 carbon atoms.

In one embodiment, R is selected from the group consisting of: C3-linear, C6-linear, C18-linear, C12-monobranched, C5-monobranched, C13-monobranched, C20-monobranched, C9-polybranched, C10- polybranched, C5-linear fluorinated, C4-linear fluorinated, C7-linear fluorinated, perfluorinated and any mixture thereof.

In one embodiment, M is a paramagnetic trivalent ion.

In one embodiment, M is selected from the group consisting of: Sc, Ti, V, Cr, Fe, Co, Ni, Ru, Rh, Os, Ac, Pr, Nd, Sm, Eu, Gd, Tb, Dy, Ho, Er, Tm, Yb, Lu, and any mixture thereof.

In one embodiment, M is Ho, Er or a mixture thereof.

In one embodiment, said oil comprises or consists of fluorinated oil.

"Aliphatic chain" includes a straight or branched hydrocarbon radical or group having at least one carbon atom including but not limited to C₃-C₆ such as: 1-propyl and 2-propyl, 1-butyl, 2-butyl, 2-methyl-1-propyl, 1,1-dimethylethyl, 1-pentyl, 2-pentyl, 3-pentyl, 2-methyl-1-butyl, 3-methyl-1-butyl, 2,2-dimethylpropyl, 1-hexyl, 2-hexyl, 3-hexyl, 2-methyl-1-pentyl, 3-methyl-1-pentyl, 4-methyl-1-pentyl, 3,3-dimethyl-1-butyl, 3,3-dimethyl-2-butyl, 2-ethyl-1-butyl and the like; C₇-C₁₂ such as: 1-heptyl, 2-heptyl, 3-heptyl, 4-heptyl, 2-methyl-1-hexyl, 4-methyl-1-hexyl, 5-methyl-1-hexyl, 1-octyl, 2-octyl, 3-octyl, 4-octyl, 6-methyl-1-heptyl, 5,5-dimethyl-1-hexyl, 2-ethyl-1-hexyl, 2-methyl-1-heptyl, 2-propyl-1-pentyl, 1-nonyl, 2-nonyl, 2-ethyl-2-methyl-1-hexyl, 4-methyl-1-octyl, 3,5,5-trimethyl-1-hexyl, 1-decyl, 2-decyl, 4-ethyl-1-octanyl, 2-methyl-1-nonyl, 4-methyl-1-nonyl, 8-methyl-1-nonyl, 1-undecyl (1-hendecyl), 2-undecyl, 7-methyl-1-decyl, 1-dodecyl, 5-dodecyl, 2-butyl-1-octyl, 10-methyl-1-undecyl and the like; C₁₃-C₁₈ such as: 1-tridecyl, 4-methyl-1-dodecyl, 11-methyl-1-dodecyl, 1-butyldecyl, 11-methyl-1-tridecyl, 1-pentadecyl, 1-hexadecyl, 2-hexyl-1-decyl, 1-heptadecyl, 14-methyl-1-hexadecyl, 15-methyl-1-hexadecyl, 1-octadecyl, 16-methyl 1-heptadecyl and the like; C₁₉-C₃₀ such as 1-nonadecyl, 2-methyl-1-octadecyl, 10-methyl-1-octadecyl, 17-methyl-1-octadecyl, 2,6,10,14-tetramethylpentadecyl, 1-eicosyl (1-arachidinyl, 1-leicosanyl), 18-methyl-1-nonadecyl, 1-heneicosyl, 19-methyl-1-eicosyl and 1-docosyl (1-behenyl), 1-tricosyl, 1-tetracosyl, 1-pentacosyl, 1-hexacosyl, 1-heptacosyl, 1-octacosyl, and the like. "Alkyl" groups may be unsubstituted or substituted. "Alkyl" groups may comprise one or more heteroatoms replacing carbons atoms, and may comprise typically 1 to 3 heteroatoms. Heteroatom are usually selected from O, S, and N.

"Alky" groups having may optionally contain one or more unsaturation sites, such as alkenyl groups or alkynyl radicals. "Alkenyl" are analogous to "alkyl" but have at least one double bond (two adjacent sp² carbon atoms). If possible, the geometry of the double bond may be trans (E) or cis (Z). Similarly, "alkynyl" have at least one triple bond (two adjacent sp carbon atoms). Unsaturated alkenyl or alkynyl may have one or more double or triple bonds, respectively, or a mixture thereof. Like "alkyl", unsaturated groups may be straight chain or branched. Examples of alkenyls include vinyl, allyl, 2-methyl-2-propenyl, cis-2-butenyl, trans-2-butenyl, and acetyl, propene, 1-butene, 2-butene, 2-methylpropene, 1-pentene, 2-petnene, 2-methyl-1-butene, 2-methyl-2-butene, 3-methyl-1-butene, 1-hexene, 2-hexene, 3-hexene, 2,3-dimethyl-1-butene, 2,3-dimethyl-2-butene, 3,3-dimethyl-1-butene, 2-dimethyl-2-butene, 2-ethyl-1-butene, 2-methyl-1-pentene, 2-methyl-2-pentene, 3-methyl-1-pentene, 3-methyl-2-pentene, 4-methyl-1-pentene, 4-methyl-2-pentene, 1-heptene, 2-heptene, 3-heptene, 3,4-dimethyl-2-pentene, 4,4-dimethyl-2-pentene, 3-methyl -2-hexene, 3-methyl -3-hexene, 4-methyl -2-hexene, 2,3-dimethyl-1-pentene, 2,3-dimethyl-2-pentene, 2,4-dimethyl-1-pentene, 2,4-dimethyl-2-pentene, 3,3-dimethyl-1-pentene, 3,4-dimethyl-1-pentene, 4,4-dimethyl-1-pentene, 4,4-dimethyl-2-pentene, 3-ethyl-1-pentene, 3-ethyl-2-pentene, 2-methyl-1-hexene, 2-methyl -2-hexene, 3-methyl-1-hexene, 4-methyl-1-hexene, 5-methyl-1-hexene, 2,3,3-trimethyl-1-butene, 1-octene, 2-octene, 3-octene, 4-octene, 2,2-diemethyl-3-hexene, 2,3-dimethyl-2-hexene, 2,3-dimethyl -3-hexene, 3-ethyl-2-methyl-1-pentene, 3-ethyl-2-methyl-pent-2-ene, 2-isopropyl-1-pentene, 2-methyl-1-heptene, 2-methyl-2-heptene, 4-methyl-2-heptene, 2,3,4-trimethyl-2-pentene, 2,4,4-trimethyl-1-pentene, 2,4,4-trimethyl-2-pentene, 3,4,4-trimethyl-2-pentene, 1-nonene, 2-nonene, 3-nonene, 4-nonene, 2,2-dimethyl-3-heptene, 3,5,5-trimethyl-1-hexene, 1 -decene, 4-decene, 5-decene, 3,7-dimethyl-1-octene, 2-methyl-1-nonene, 1-undecene,trisisobutylene, 2,2,4,6,6-pentamethyl-3-heptene, 1-dodecene, 2-methyl-1-undecene, 1 -tridecene, 1,1-dineopentylethylene, 1-tetradecene, 7-tetradecene, 1-pentadecene, 1-hexadecene, 1-heptadecene, 8-heptadecene, 1-octadecene, 1-nonadecene, 1-eicosene, 9-heneicosene, 1-docosene and the like.

As used herein, alkyl" also includes mixed alkenyl and alkynyl groups.

"alkyl may be linear or branched (mono-branched or poly-branched).

The mono-branched or poly-branched, saturated C16-C22 fatty acids comprise alkyl side branches which are directly attached to a carbon atom of the longest linear chain

"Linear" means that the aliphatic chain does not comprise any branching points in carbon-carbon or carbon-heteroatom sites.

"Monobranched" means that the aliphatic chain comprises a linear, saturated or unsaturated aliphatic chain substituted by only one linear saturated or unsaturated aliphatic chain.

Examples of monobranched aliphatic chains are:
2-butyl-1-octyl
3-methyl-1-butyl
2-octyl-1-dodecyl
2-Hexyl-1-decyl
2-Decyl-1-tetradecyl
2-Octyl-1-dodecyl
2-Propyl-1-pentyl
2-Pentyl-1-nonyl
2-hexadecyl
2-dodedecyl

"Polybranched" means that a linear, saturated or unsaturated aliphatic chain comprises two or more linear or branched, saturated or unsaturated aliphatic chains. Examples of polybranched aliphatic chains are:
3,7-dimethy-1-octyl
3,5,5-trimethyl-1-hexyl

Examples of R in molecules of formula (I) or (II) are:
1-octadecyl
1-hexyl
2-butyl-1-octyl
3-methyl-1-butyl
2-octyl-1-dodecyl
3,7-dimethy-1-octyl
3,5,5-trimethyl-1-hexyl
C12 or C13-alkyl or a mixture thereof
2,2,3,3,4,4,5,5-octafluoro-1-pentyl
2,2,3,3,4,4,4-heptafluoro-1-butyl
1H,1H-tridecafluoro-1-heptyl.

"Alkyl" also includes cyclic groups having at least three carbons optionally containing at least one unsaturation site, and being known as cycloalkyl radicals.

The term "cycloalkyl" as used herein means a monocyclic or polycyclic hydrocarbyl group. Illustrative examples of a cycloalkyl radical include cyclopropyl, cycloheptyl, cyclooctyl, cyclodecyl, cyclobutyl, adamantyl, norpinanyl, decalinyl, norbornyl, cyclohexyl, and cyclopentyl. Cycloalkyl groups may be unsubstituted or substituted. Other examples are rings in which 1 to 3 heteroatoms replace carbons. Such groups are termed "heterocyclyl" and examples are oxiranyl, pyrrolidinyl, piperidyl, tetrahydropyran, and morpholine.

In one embodiment, saturated or unsaturated aliphatic chains according to the invention may be substituted by one or more fluorine atoms. As used herein the term "substituent" or "substituted" means that a hydrogen radical on a molecule or group is replaced with a fluorine atom.

In one embodiment, saturated or unsaturated aliphatic chains according to the invention may be substituted by one or more atom or group of atoms selected from the group consisting of halogen, notably chloro, iodo, bromo, or fluoro, cyano hydroxyl; alkoxy; nitro; thiol; thioether; imine; amido; phosphonato; phosphine; carboxyl; thiocarbonyl; sulfonyl; sulfonamide; ketone; aldehyde; ester; oxygen; haloalkyl (e.g., trifluoromethyl); cycloalkyl, which may be monocyclic or fused or non-fused polycyclic (e.g., cyclopropyl, cyclobutyl, cyclopentyl, or cyclohexyl), or a heterocycloalkyl, which may be monocyclic or fused or non-fused polycyclic, monocyclic or fused or non-fused polycyclic aryl or heteroaryl; amino (primary, secondary, or tertiary); CO₂CH₃; CONH₂; OCH₂CONH₂; NH₂; SO₂NH₂; OCHF₂; CF₃; OCF₃.

In one embodiment, M is Ho.

In one embodiment, M is Er.

In one embodiment, M is Eu.

In one embodiment, M is Gd.

In one embodiment, M is Tb.

In one embodiment, M is Dy.

In one embodiment, M is Tm.

In one embodiment, M is Ti.

In one embodiment, M is V.

In one embodiment, M is Fe.

In one embodiment, M is Cr.

In one embodiment, X is O.

In one embodiment, X is S.

In one embodiment, X is N.

Examples of molecules of formula (I) are:
Ho-DTPA-(2-butyl-1-octanol)₂
Ho-DTPA-(3,7-dimethy-1-octanol)₂
Ho-DTPA-(2-octyl-1-dodecanol)₂
Ho-DTPA-(hexanol)₂
Er-DTPA-(3,5,5-trimethyl-1-hexanol)₂

### Composition

In one embodiment, said oil is a paraffin oil or an alcohol having a linear or branched, saturated or unsaturated aliphatic chain.

In one embodiment, said composition is liquid.

Advantageously, the chemical nature of the side chains of the DTPA cage should be solvophilic with the oil used for the composition itself. The composition could be also defined as a mixture of structure (I) and a carrier medium.

In one embodiment, said composition is a paramagnetic liquid.

In one embodiment, said composition is an organic liquid, and for example an oil.

The term "oil" as used herein refers to any material which is substantially insoluble in water or water-immiscible.

In one embodiment, "water-immiscible" means that said molecule or composition has a solubility in water of less than 0.1% w/w at 25°C, 101325 Pa, and pH 7.

Water-immiscibility can be assayed according to the following procedure: a first aliquot of immiscible medium of 0.01% w/w is added to water and vigorously shaken. The turbidity (at a non-absorbing wavelength) of the solution is measured immediately. If turbidity is detected after the first aliquot the medium is immiscible. If the turbidity of the solution is the same as or equivalent to the turbidity of pure water, the specific composition is soluble. Another aliquot of immiscible medium is added (i.e., 0.02% w/w in total) and the procedure of shaking and turbidity measurement is repeated. The latter step is repeated until significant turbidity is detected. The threshold concentration of immiscible medium in water where turbidity is detected is defined as the solubility limit. Above the solubility limit the medium is water-immiscible, below it is water-miscible.

Suitable oil components include, but are not limited to, natural oils, hydrocarbons such as mineral oil and hydrogenated polyisobutene, fatty alcohols; fatty esters; fatty diesters; fatty triesters, silicones and any mixture thereof.

In one embodiment, said oil comprises or consists of a paraffin (mineral) oil.

In one embodiment, said oil comprises or consists of an alcohol having a linear or branched, saturated or unsaturated aliphatic chain having from 6 to 30 carbon atoms.

In one embodiment, said oil comprises or consists of a linear or branched, saturated or unsaturated aliphatic chain identical to R.

In one embodiment, said oil comprises or consists of fluorinated oil.

In one embodiment, said oil comprises or consists of a perfluorinated oil.

In one specific embodiment, said oil comprises or consists of perfluorodecalin.

In one embodiment, said oil comprises or consists of silicone oil.

In one embodiment, said oil comprises or consists of vegetable oil.

In one embodiment, said composition comprises one or more surfactants. Advantageously, a surfactant helps to lower the surface tension at the liquid-liquid interface of the composition and a second immiscible liquid.

In one embodiment, said composition is transparent.

In one embodiment, said composition is a non-ionic composition.

In one embodiment, said composition is a transparent non-ionic magnetic composition.

In one embodiment, said composition is a transparent non-ionic magnetic oil.

In one embodiment, said composition improves anti-fouling and low-shearing properties of a device or a method for circulating a less-paramagnetic liquid into an enclosing more paramagnetic liquid or for circulating a more-paramagnetic liquid into an enclosing less paramagnetic liquid. Such improvement can be tested in comparison with a ferrofluid consisting of dispersed magnetite nanoparticles in an oil medium.

In one embodiment, said composition is immiscible with nearly all solvents (aqueous, oily, emulsions, etc.), where immiscibility can be typically assayed as described above.

In one embodiment, said composition comprises from 1 to 50% by weight of said oil relative to the total weight of the composition.

In one embodiment, said composition comprises from 1 to 40% by weight of said oil relative to the total weight of the composition.

In one embodiment, said composition comprises from 1 to 30% by weight of said oil relative to the total weight of the composition.

In one embodiment, said composition comprises from 1 to 20% by weight of said oil relative to the total weight of the composition.

In one embodiment, said composition comprises from 1 to 10% by weight of said oil relative to the total weight of the composition.

In one embodiment, said composition comprises 50% or more by weight of at least one molecule of structure (I) relative to the total weight of the composition.

The invention also relates to a method for the synthesis of a molecule having the general structure (II).

The invention relates in particular to a method for the synthesis of a molecule having the following general structure (II): 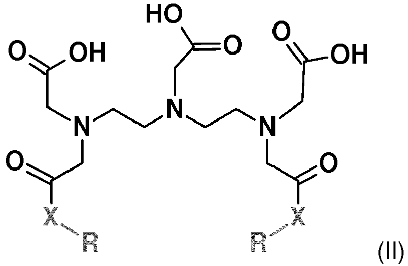 wherein
X is O, N, or S ;
R represents at each occurrence the same or different and independently linear or branched, saturated or unsaturated aliphatic chain, non-fluorinated, partially fluorinated or perfluorinated; and
M is a trivalent paramagnetic ion,
said method comprising:
(i) placing diethylenetriaminepentaacetic acid (DTPA) or a derivative thereof in contact with a compound of formula:
   RXH if X is O or S, or
   RXH₂ if X is N;
(ii) reacting the mixture of step (i) to obtain the molecule having the general structure (II).

The method for synthetizing molecules of formula (II) comprises placing DTPA or a derivative thereof such as for example DTPA dianhydride, in contact with a compound of formula RXH in a solvent, notably for dissolving DTPA and RXH compound in said solvent. If needed, the mixture can be heated to dissolve DTPA and RXH in the solvent.

In one embodiment, the heating takes place by microwave irradiation.

In one embodiment, the solvent is pyridine.

In one embodiment, the solvent is N,N-Dimethylformamide (DMF).

In one embodiment; DTPA or a derivative thereof such as for example DTPA dianhydride, is dispersed or dissolved in a solvent and then RXH is added to the solvent comprising DTPA or a derivative thereof.

Advantageously, the synthesis comprises a step of obtaining a homogeneous solution of molecule of formula (II).

In one embodiment, the method of synthesis comprises removing the solvent. Methods for removing solvents are known by skilled persons.

Advantageously, the synthesis is performed under conditions of temperature, concentration and reaction time avoiding unwanted triester derivatives.

In one embodiment, the compound RXH is ROH.

In one embodiment, the compound RXH is RSH.

In one embodiment, the compound RXH is RNH₂.

In one embodiment, resulting compounds of structure (II) can be dried. Drying of methods are known by skilled persons.

The invention also relates to a method for the synthesis of a molecule having the general structure (I).

The invention relates in particular to a method for the synthesis of a molecule having the following general structure (I): 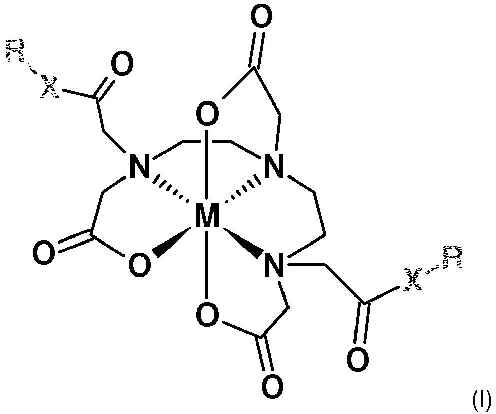 wherein
X is O, N, or S ;
R represents at each occurrence the same or different and independently linear or branched, saturated or unsaturated aliphatic chain, non-fluorinated, partially fluorinated or perfluorinated; and
M is a trivalent paramagnetic ion,
said method comprising placing a molecule having the general structure (II) in contact with a salt of M.

In one embodiment, the method comprises dissolving a molecule of structure (II) in a solvent.

In one embodiment, said solvent is THF.

In one embodiment, said solvent is an alcohol, for example a primary alcohol such as for example ethanol.

In one embodiment, said solvent is a mixture of an alcohol and water, for example 1:1 ethanol/water mixture.

In one embodiment, a salt of M is MCl₃.

In one embodiment, a salt of M is M(OAc)₃.

In one embodiment, a salt of M is M(NO₃)₃.

In one embodiment, a molecule of structure (II) is dissolved in THF, followed by the addition of MCl₃ and an amine base, such as for example trimethylamine.

Advantageously, the addition of the amine solvent causes the complete solubilization of MCl₃.

In one embodiment, molecule (I) is prepared by maintaining the reaction mixture under reflux of the solvent.

In one embodiment, the solvent is removed to get the molecule of structure (I).

In one embodiment, a molecule of structure (II) is placed in contact with an alcoholic solvent, such as for example ethanol and an aqueous solution of NaOAc is added, for example at room temperature (20°C) followed by the addition of M(OAc)₃. The mixture is dissolved in an alcoholic/water mixture containing a buffer, for example an acetate buffer

Advantageously, the synthesis molecules of structure (I) are prepared by placing a molecule of structure (II) in contact with a base, for example NaOH. In one embodiment MCl₃ is added to a solution containing molecule structure (II) and the base.

In one embodiment an alcoholic/water mixture, for example ethanol/water, is used as the solvent.

In one embodiment, the solvent is evaporated.

Advantageously, the solvents are removed after the desired reaction.

Methods for removing the solvents are known by skilled persons.

In one embodiment, structures of molecule (I) are dried. Drying of methods are known by skilled persons.

### Use/applications

Advantageously, molecules of structure (I) present magnetic properties.

Accordingly, such molecules of structure (I) can be used in paramagnetic compositions or for applications as paramagnetic compositions.

The molar magnetic susceptibility of the composition is identical or similar to that of the specific paramagnetic ion used. For example, a composition containing a concentration of 1M of Ho-DTPA-R₂ has nearly exactly the same susceptibility as a 1M solution of HoCl₃ in water (i.e., as Ho³⁺ and Cl⁻ hydrated ions).

The invention also relates to a method for preparing a paramagnetic composition.

In one embodiment, at least one molecule of structure (I) is dissolved in a solvent, for example dichloromethane, preferably to get a homogenous solution. An oil, for example a fatty alcohol, is added to this solution. In one embodiment, the solvent, for example dichloromethane, is evaporated. Such methods for evaporating solvents are known by skilled persons.

In one embodiment, said composition is used in a microfluidic device, a magnetically actuated fluid valve, a magnetically actuated peristaltic pump, an anti-fouling device, an anti-clogging device, a propulsion device, a heat exchanger, an extractor, a desalinator, or a mixing device.

In one embodiment, said composition is for pharmaceutical or diagnostic applications.

In one embodiment, said composition is for biological applications.

In one embodiment, said composition is for microreactor applications.

Advantageously, other properties of the used ion will also be propagated in the composition, such as for example, a fluorescent (paramagnetic) ion (e.g., Er³⁺) will lead to a fluorescent magnetic composition.

In one embodiment, said composition is for magneto-optic applications, where the Faraday effect causes rotation of the plane of light polarization. The latter effect can be used as a Faraday rotator, Faraday isolator, or in optical isolators.

In one embodiment, said composition is for lensing applications, where the composition is layered with an immiscible secondary liquid, resulting in a liquid lens. The shape of the latter lens can be modified by applying an arbitrary external magnetic field.

Other aims, characteristics and advantages of the invention will appear clearly to the person skilled in the art upon reading the explanatory description which makes reference to the Examples which are given simply as an illustration and which in no way limit the scope of the invention.

The Examples make up an integral part of the present invention, and any characteristic which appears novel with respect to any prior state of the art from the description taken in its entirety, including the Examples, makes up an integral part of the invention in its function and in its generality.

Thus, every example has a general scope.

Furthermore, in the Examples, all percentages are given by weight, unless indicated otherwise, temperature is expressed in degrees Celsius unless indicated otherwise, and the pressure is atmospheric pressure, unless indicated otherwise.

### EXAMPLES

### EXAMPLE 1 - Synthesis of the DTPA-based ligand, i.e. molecules of structure (II)

### EXAMPLE 1.1 (using pyridine as the reaction solvent)

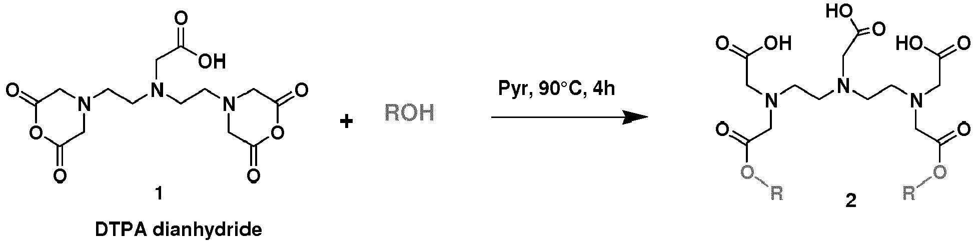

DTPA dianhydride and the alcohol, ROH (2 eq.), are dissolved in pyridine (0.1-0.2 M) and the mixture is heated to 90°C and left under stirring 4h. After that, pyridine is removed by rotary evaporation, followed by the addition of methanol and further evaporation to dryness.

ROH used are recited in Table 1.

This method worked very well when using stearyl alcohol (1-octadecanol) as ROH.

### EXAMPLE 1.2 (in DMF using microwave synthesis)

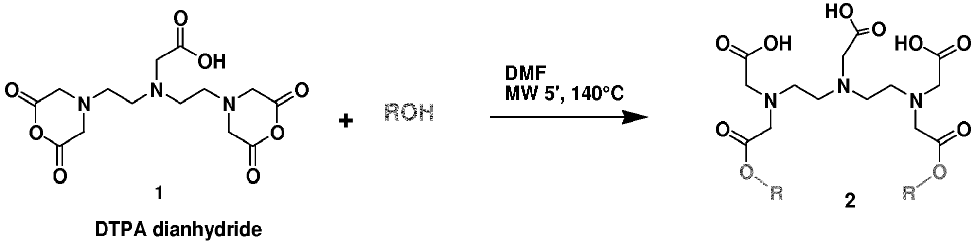

DTPA dianhydride is dispersed in DMF (0.1-0.2 M) in a pressure tight vial and then the alcohol, ROH (2.2 eq.) is added. The suspension is heated to 140°C by MW irradiation for 5 min under continuous stirring, thus obtaining a yellowish homogeneous solution. Afterwards, the solvent is removed by rotary evaporation affording **2** as a sticky solid.

ROH used are recited in Table 1.

### EXAMPLE 1.3 (using DMF as the reaction solvent)

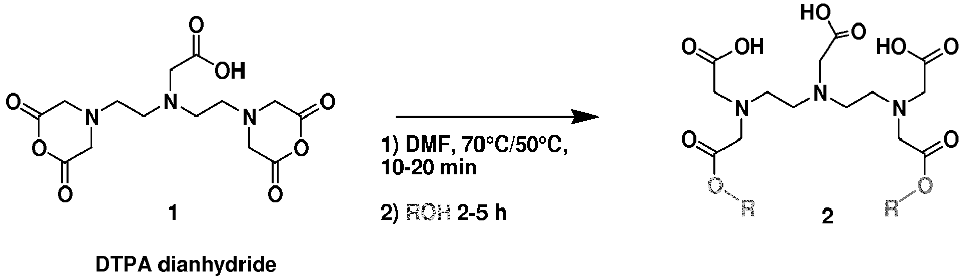

DTPA dianhydride is dispersed in dry dimethylformamide (DMF) under argon atmosphere (typical concentrations 0.05-0.2 M). The obtained suspension is heated to 70-75°C under stirring, till complete solubilization is observed (usually it takes 5-20 min, depending on actual DTPA concentration and total amount). After that, the long chain alcohol, ROH, is added dropwise to the above solution and the resulting homogeneous mixture is left under stirring at 50°C (typically 3-5h). The completeness of the reaction is monitored by liquid chromatography/mass-spectrometry (LC-MS). Afterwards, the solvent is removed (rotary evaporation + high vacuum pump) to give **2** as a sticky yellowish solid.

ROH used are recited in Table 1.

This method was successfully scaled up to 2g of starting DTPA dianhydride.

**Table 1**

| **ROH** | **Kind of chain in molecule of structure (2)** | **Method used** |
|---|---|---|
| 1-octadecanol | C18-linear | Example 1.1 |
| 1-propanol | C3-linear (short) | Example 1.2 |
| 1-hexanol | C6-linear (short) | Example 1.2/1.3 |
| 2-butyl-1-octanol ISOFOL 12® | C12-monobranched | Example 1.3 |
| 3-methyl-1-butanol | C5-monobranched (short) | Example 1.3 |
| 2-octyl-1-dodecanol | C20-monobranched | Example 1.3 |
| 3,7-dimethy-1-octanol | C10-polybranched | Example 1.3 |
| 3,5,5-trimethyl-1-hexanol | C9-polybranched | Example 1.3 |
| ISALCHEM 123® | Mixture of monobranched primary C12-C13-alcohols | Example 1.3 |
| 2,2,3,3,4,4,5,5-Octafluoro-1-pentanol | C5-linear fluorinated | Example 1.3 |
| 2,2,3,3,4,4,4-Heptafluoro-1-butanol | C4-linear fluorinated | Example 1.3 |
| 1 H,1H-Tridecafluoro-1-heptanol | C7-linear fluorinated | Example 1.3 |

### EXAMPLE 2 - Synthesis of the neutral metal complex, i.e. molecules of structure (I)

### EXAMPLE 2.1 (in THF at reflux)

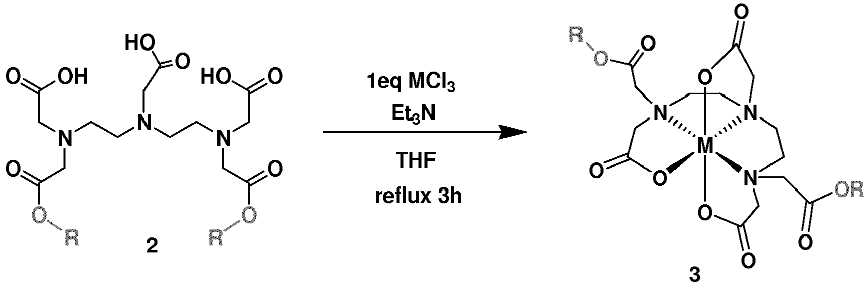

Ligand **2** was dissolved in THF (0.03M), followed by the addition of solid MCl₃ (1 eq. with respect to **2**) and trimethylamine (3 eq.). The addition of the latter causes the complete solubilization of the inorganic salt in the reaction mixture, yielding a homogeneous solution (e.g. pinkish when using HoCl₃), which is heated to reflux and kept under stirring 3h. During this time, a white precipitate appears which is filtered off, while the organic filtrate is evaporated to dryness giving **3** as a sticking solid.

This method has been used successfully to prepare Ho-DTPA-(1-octadecanol)₂.

### EXAMPLE 2.2 (in acetate buffer)

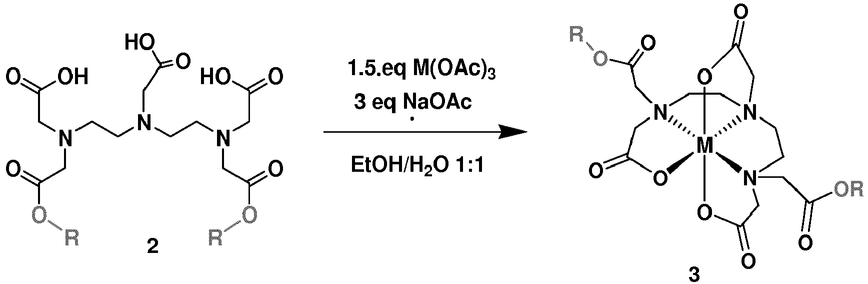

To a stirred solution of the ligand **2** in ethanol (0.2-0.4 M), an aqueous 1 M solution of NaOAc is added at RT (3 eq. with respect to **2**), followed by the addition of the acetate salt of a rare-earth metal, M(OAc)₃ (1.5 eq. with respect to **2**), dissolved in the proper amount of water so to have a final 0.1-0.2 M solution of the complex of **3** in acetate buffer (AcO⁻/AcOH, 1.5:1), in ethanol/water 1:1. The resultant homogeneous mixture is left under stirring at RT for 0.5-1h. After that, the solution is concentrated by rotary evaporation, without heating (to eliminate EtOH), followed by addition of water and extraction using dichloromethane. The organic phase was dried over MgSO₄ and concentrated to give the complex **3**, usually as a glassy solid.

When using short alcohols with less than 6 carbon atoms, the resulting complex **3** is water soluble, e.g. it remains in water during the water/dichloromethane extractions. Because of that, and for the sake of stability, it would be better to consider only water insoluble complexes (i.e. bearing tails longer than 6 C atoms) in the composition of transparent non-ionic magnetic oil.

### EXAMPLE 2.3 (using NaOH as base)

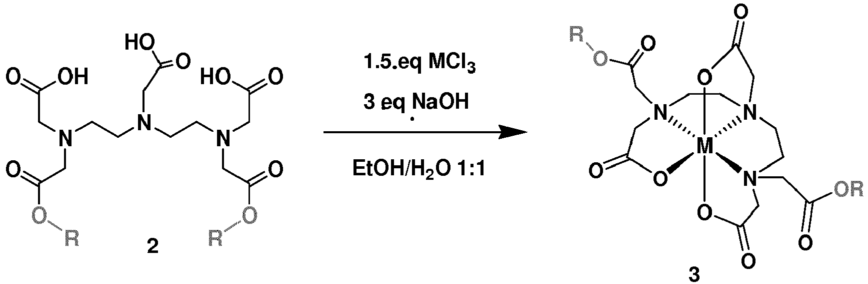

At a stirred solution of the ligand **2** in ethanol (0.2M), an aqueous 1 M solution of NaOH is added dropwise at room temperature RT (3 eq.), followed by the addition of the chloride salt of a rare-earth metal, MCl₃ (1.5 eq. with respect to **2**), dissolved in the proper amount of water so to have a final 0.1 M solution of the complex of **3** in ethanol/water 1:1. The resultant homogeneous mixture is left stirring at RT, untill a viscous oil (which responds to magnetic fields) separates out from the water phase (typically 0.5-1 h). After that, the solution is concentrated by rotary evaporation (mainly to eliminate EtOH) and the oil is extracted from the water phase with dichloromethane. The combined organic extracts are dried over MgSO₄ and rotary evaporated to give the complex **3** as a glassy solid.

This method was particularly successful to prepare the following complexes:
Ho-DTPA-(2-butyl-1-octanol)₂
Er-DTPA-(3,5,5-trimethyl-1-hexanol)₂

### EXAMPLE 3 - UV-Vis characterization of molecules of structure (I)

UV-Vis characterization of molecules of structure (I) has been checked.

For example, the UV-Vis spectrum of Er-DTPA-(3,5,5-trimethyl-1-hexanol)₂ in methanol showed the characteristic absorption peaks of Er³⁺, whose molar extinction was in a very good agreement with that previously reported [Sastri, Vinny R., et al. Modern aspects of rare earths and their complexes. Elsevier, 2003], thus confirming the success of the complexation.

### EXAMPLE 4 - Measurement of the magnetic susceptibility

For the prepared molecules of structure (I) in table 2 below, the paramagnetic properties have been evaluated by the Evans method [D.F. Evans, J. Chem. Soc. 2003 (1959)]. This is based on nuclear magnetic measurements (NMR), and relies on the fact that the chemical shift (position) of the ¹H NMR signals of a molecule depend on the bulk susceptibility of the medium. In particular, the resonance line of the residual proton of CDCl₃ (i.e. the NMR solvent) was used as a reference, by comparing its chemical shift for the pure CDCl₃ and for a solution containing the molecules of structure (I).

**Table 2**

| molecule of structure (I) | **χₘₒₗ** (cm³ mol⁻¹) | **χₘₐₛₛ** (cm³ g⁻¹) /10-5 |
|---|---|---|
| Ho-DTPA-(2-butyl-1-octanol)₂ | 0.0350 | 4.98 |
| Ho-DTPA-(3,7-dimethy-1-octanol)₂ | 0.0422 | 5.05 |
| Ho-DTPA-(2-octyl-1-dodecanol)₂ | 0.0415 | 3.72 |
| Ho-DTPA-(hexanol)₂ | 0.0437 | 6.04 |
| Ho-DTPA-( ISALCHEM 123)₂ | 0.0385 | 4.25 |
| Ho(AcO)_{3 *}nH₂O (inorganic salt as reference) | 0.037 | 11.0 |

| | | |
|---|---|---|
| Where χₘₒₗ, χₘₐₛₛ are respectively the molar and mass susceptibility with (χₘₒₗ = MW*χₘₐₛₛ) | | |

As expected the mass susceptibility of the different complexes (molecules of structure (I)) vary depending on mass ratio between the paramagnetic ion (e.g. Ho³⁺) and the organic DTPA-ligand (thus, it is smaller for the bigger ligands). However, the calculated molar susceptibility (i.e. normalized for the molecular weight of the complex, or averaged molecular weight in the case of side chain mixtures, e.g. for Ho-DTPA-(ISALCHEM 123)₂) are very similar for the different complexes, and comparable with that of the inorganic salt Ho(AcO)₃, as well as of the order of magnitude expected for Ho³⁺ [Coey, John MD. Magnetism and magnetic materials. Cambridge University Press, 2010]

### EXAMPLE 5 - Preparation of paramagnetic oils

### EXAMPLE 5.1 (composition with a fatty alcohol)

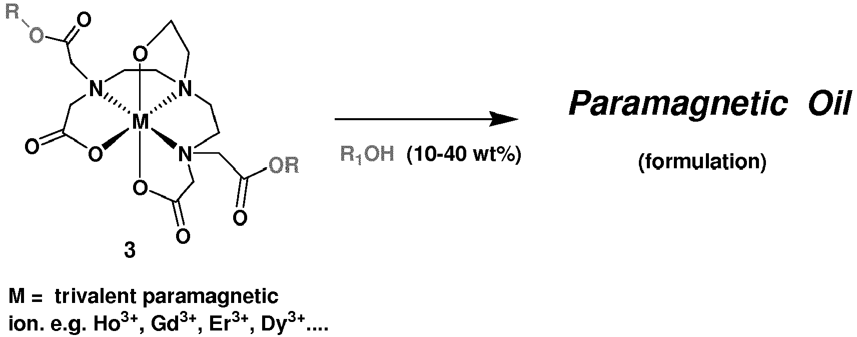

The complex **3** (molecules of structure (I)) is dissolved in the minimum amount of dichloromethane, so to have a homogeneous solution, after that a fatty alcohol (e.g. isofol® 12) is added (typically 10-40 wt%), and the resultant mixture rotary evaporated under high vacuum to completely eliminate the volatile dichloromethane, resulting in the target homogeneous transparent paramagnetic oil.

The added alcohol R₁OH can be the same or different than those used for the preparation of molecule **2** (ROH).

### EXAMPLE 5.2 (composition with mineral oil)

The composition is prepared according to example 5.1 but using paraffin (mineral) oil (typically 10-30 wt%) as co-component instead of the fatty alcohol.

This method is particularly suitable when using complexes with long chains (preferably more than 8 carbon atoms). This method was notably tested with Ho-DTPA-(1-octadecanol)₂ yielding a homogeneous transparent fluid reacting to magnetic fields.

### EXAMPLE 6 (magneto optic applications of composition)

The composition is prepared according to example 5.1 but using isofol 12 to obtain a weight fraction of 23 % w/w of isofol 12, where Tb³⁺ is used as the paramagnetic ion in structure (I). The Verdet coefficient was determined to be 17.1 rad T⁻¹ m⁻¹ at 410 nm wavelength, and 8.0 rad T⁻¹ m⁻¹ at 600 nm. As a control a 1 M TbCl3 aqueous solution was measured using the same method resulting in a Verdet coefficient of 14.1 rad T⁻¹ m⁻¹ at 410 nm wavelength, and 4.9 rad T⁻¹ m⁻¹ at 600 nm in agreement with literature.

### EXAMPLE 7 (magnetic lensing of composition)

The composition is prepared according to example 6, but using Ho³⁺ as the paramagnetic ion in structure (I). Equal amounts of water and composition are layered on top of each other (water being the bottom layer) in a cylindrical vial. The latter results in a layer of composition, which acts as a biconcave lens through which an image can be focussed under an optical stereoscope by applying an external magnetic field (using permanent magnets). In addition, the magnetic liquid lens can be deformed asymmetrically by applying an asymmetrical external magnetic field.

## Claims

1. A composition comprising an oil and at least one molecule having the following general structure (I): 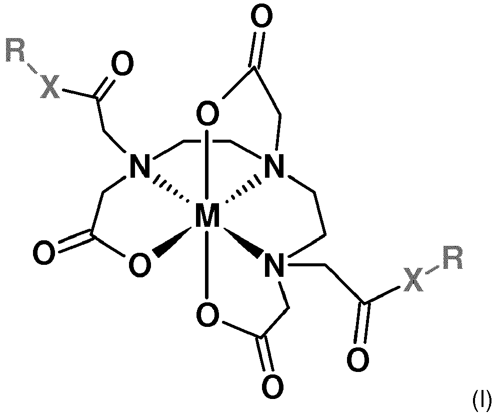 wherein X is O, N, or S ; R represents at each occurrence the same or different and independently linear or branched, saturated or unsaturated aliphatic chain, non-fluorinated, partially fluorinated or perfluorinated; and M is a trivalent paramagnetic ion.

2. The composition according to claim 1, wherein R comprises from 3 to 30 carbon atoms.

3. The composition according to claim 1 or 2, wherein R comprises from 6 to 30 carbon atoms.

4. The composition according to any one of claims 1 to 3, wherein R is selected from the group consisting of: C3-linear, C6-linear, C18-linear, C12-monobranched, C5-monobranched, C13-monobranched, C20-monobranched, C9-polybranched, C10-polybranched, C5-linear fluorinated, C4-linear fluorinated, C7-linear fluorinated, perfluorinated and any mixture thereof.

5. The composition according to any one of claims 1 to 4, wherein M is a paramagnetic trivalent ion.

6. The composition according to any one of claims 1 to 5, wherein M is selected from the group consisting of: Sc, Ti, V, Cr, Fe, Co, Ni, Ru, Rh, Os, Ac, Pr, Nd, Sm, Eu, Gd, Tb, Dy, Ho, Er, Tm, Yb, Lu, and any mixture thereof.

7. The composition according to any one of claims 1 to 6, wherein M is Ho, Er or a mixture thereof.

8. The composition according to any one of claims 1 to 6, wherein said oil comprises or consists of perfluorinated or partially fluorinated oil.

9. The composition according to claim 8, wherein said oil is a paraffin oil or an alcohol having a linear or branched, saturated or unsaturated aliphatic chain.

10. The composition according to claim 8 or 9, wherein said composition is liquid.

11. The composition according to any one of claims 8 to 10, wherein said composition comprises one or more surfactants.

12. The composition according to any one of claims 8 to 11, wherein said composition comprises from 1 to 50% by weight of said oil relative to the total weight of the composition.

13. A method for the synthesis of a molecule having the following general structure (II): 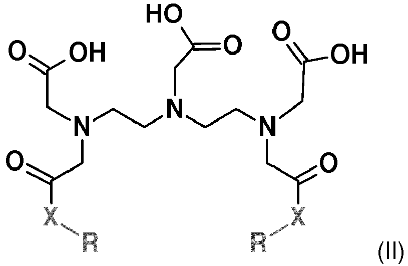 wherein
X is O, N, or S ;
R represents at each occurrence the same or different and independently linear or branched, saturated or unsaturated aliphatic chain, non-fluorinated, partially fluorinated or perfluorinated; and
M is a trivalent paramagnetic ion,
said method comprising:
(i) placing diethylenetriaminepentaacetic acid (DTPA) or a derivative thereof in contact with a compound of formula:
RXH if X is O or S, or
RXH₂ if X is N;
(ii) reacting the mixture of step (i) to obtain the molecule having the general structure (II).

14. A method for the synthesis of a molecule having the following general structure (I): 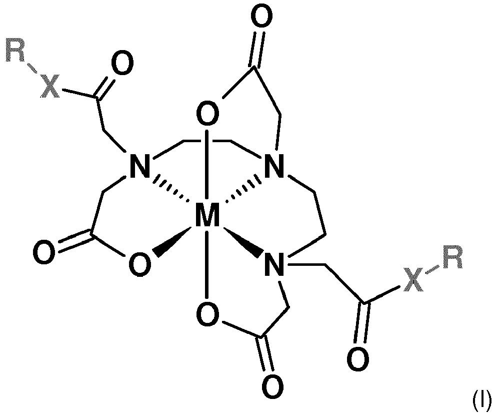 wherein
X is O, N, or S ;
R represents at each occurrence the same or different and independently linear or branched, saturated or unsaturated aliphatic chain, non-fluorinated, partially fluorinated or perfluorinated; and
M is a trivalent paramagnetic ion,
said method comprising placing a molecule having the general structure (II) in contact with a salt of M.

15. Use of a molecule having the following general structure (I) according to any one of claims 1 to 7 or of a composition according to any one of claims 8 to 12, for applications as paramagnetic composition.
